# EUROPEAN PATENT APPLICATION

(11) **EP 1 897 565 A1**
(43) Date of publication of application: **12.03.2008**
(21) Application number: 07253606.3
(22) Date of filing: 11.09.2007
(51) Int. Cl.: A61L 9/04

(54) **Odour and bird control formulation**

(30) Priority: 11.09.2006 GB 0617824
(71) Applicant: UK Control Limited, Carnforth Lancashire LA6 2PL (GB)
(72) Inventor: Whittaker, Ian, Carnforth, Lancashire LA6 2PL (GB)
(74) Representative: Wilson Gunn

(57) **Abstract**

A control formulation that is suitable for odour control and for use as bird control/bird repellent, the control formulation comprising at least one active agent, at least one solvent, at least one surfactant, and at least one gum, wherein the active agent is an anthranilic acid derivative, acetophenone derivative, veratroyl derivative, cinnamic acid derivative, or alkyl phenyl acetate derivative, and the formulation is adapted for odour control and bird control.

## Description

The present invention relates to formulations for use as an odour control formulation, and particularly, but not exclusively, to formulations having odour control properties for use on landfill waste sites. The present invention also relates to formulations for use as bird control/bird repellent formulations.

The problem of providing control of odour is one that is well known. The problem is particularly evident at landfill waste sites where degrading rubbish often causes unpleasant odours. These odours which often emanate from landfill waste sites can be very unpleasant, and are undesirable to both the people who work at the site, and additionally to people who neighbour the site. In particular, the unpleasant odours can be very detrimental to neighbouring populated areas, such as neighbouring residential areas.

Prior attempts to control odours have included application of chemical formulations. However, such formulations often require continual spraying and formation of aerosol fogs above the site to be treated. The formed aerosol fog can drift in to neighbouring sites, such as residential or commercial areas, and this is very undesirable.

Additionally the problem of providing control of birds and an ability to repel birds is also well know. Again, this problem is particularly evident at landfill waste sites at which large flocks of scavenger birds can often be observed. The large flocks of birds can often represent a safety problem for people working on a landfill site, and additionally may represent a health risk due to diseases carried by the birds.

The present invention seeks to provide a control formulation, which has improved odour control properties and bird repellent properties in comparison to prior attempts as described herein. The present invention further seeks to provide a method of making the control formulation having improved odour control and bird repellent properties.

According to a first aspect of the present invention there is provided a control formulation, wherein the formulation comprises;
at least one active agent;
at least one solvent;
at least one surfactant; and
at least one gum,
wherein the active agent is an anthranilic acid derivative, acetophenone derivative, veratroyl derivative, cinnamic acid derivative, or alkyl phenyl acetate derivative, and the formulation is adapted for odour control and bird control.

It has been found that the selection and combination of the components of the present invention results in a formulation which has improved odour control properties in comparison to conventional systems.

As described previously, due to the formulation of conventional systems, application is often by continual spraying or creation of aerosol fogs. It has surprisingly been found that the improved formulation can be applied directly to landfill waste sites, and does not have to be applied in the form of continual aerosols or sprays which could drift on to neighbouring areas, such as commercial or residential areas.

Additionally, it has been found that the selection and combination of the components of the present invention can provide bird repellent and bird control properties, which can aid in bird control at, for example, landfill waste sites.

The active agent is selected from;
anthranilic acid derivatives - including ester derivatives of anthranilic acid in which the phenyl group may be substituted with radicals such as alkyls, hydroxyl, alkyl, alkenyl, alkynyl, alkoxyl, aryl, aryloxyl, aralkyloxyl, aralkyl, halo, amino, nitro, or cyano radicals;
acetophone derivatives - including substituted acetophones in which the phenyl group may be substituted with radicals such as alkyls, hydroxyl, alkyl, alkenyl, alkynyl, alkoxyl, aryl, aryloxyl, aralkyloxyl, aralkyl, halo, amino, nitro, or cyano radicals;
veratroyl derivatives - including veratroyl amines, and veratroyl amides;
cinnamic acid derivatives - including derivatives whereby the hydroxyl group is substituted with radicals such as hydrogen, amino, halo, cyano, alkyl, or alkenyl radicals; and
alkyl phenyl acetate derivatives - wherein the alkyl can be any suitable linear or branched alkyl.

Suitable active agents, by way of example, include methyl anthranilate, methyl phenyl acetate, ethyl phenyl acetate, ortho-amino acetophenone, 2-amino-4,5-dimethyl acetophenone, veratroyl amine, dimethyl anthranilate, cinnamic aldehyde, cinnamamide, cinnamic acid, and combinations thereof.

More preferably, the active agent is an ester of anthranilic acid.

Most preferably, the active agent is methyl anthranilate.

Methyl anthranilate is available commercially from Aldrich Chemical Co. of Milwaukee USA, or Jiangsu Sainte K Corporation of Kunshan China.

The active agent may be used alone or in any suitable combination.

The active agent may be present in the range 30 wt.% to 2 wt.% of the control formulation. More preferably, the active agent is present in the range 25 wt.% to 5 wt.%. Most preferably, the active agent is present in the range 20 wt.% to 15 wt.%.

The solvent may be any suitable solvent. It will be understood that this would include any suitable solvent adapted to solvate the components of the control formulation of the present invention.

The solvent may comprise at least one oil component. The oil component may be any suitable oil.

Suitable oil components include those based upon terpenes or naturally occurring extracted oils.

Suitable groups of oil components, by way of example, include hemiterpenes, monoterpenes, sesquiterpenes, diterpenes, sesterterpenes, triterpenes, tetraterpenes, polyterpenes, tree extracted oils, vegetable oils, or any combination thereof.

Preferred terpenes, by way of examples, may be isoprene, geraniol, alpha-pinene, farnesol, cembrene, taxadiene, squalene, lycopene, di-pentene, beta-pinene, caryophyllene, p-cymene, carotenes, limonene, or any combination thereof.

A suitable tree extracted oil may be pine oil.

The solvent may be used alone or in any suitable combination.

Particularly preferred oils components are limonene (as a component of orange oil) and pine oil, either alone or in combination.

The at least one solvent may be present in the range 40 wt.% to 2 wt.% of the control formulation. More preferably, the solvent is present in the range 30 wt.% to 10 wt.%.

The surfactant may be any suitable surfactant. It will be understood that this would include any suitable surfactant adapted to lower the surface tension of liquid.

Surfactants, by way of example, include those classified as;
**■** anionic - including, by way of example, those based upon sulphate, sulphonate, carboxylate anions, soaps, and fatty acid salts;
**■** cationic - including those based upon quaternary ammonium salts;
**■** non-ionic surfactants - including those based upon oxides, ethoxylates, fatty acids, and cocamid derivatives;
**■** zwitterionic (amphoteric) surfactants - such as those based upon betaines, and alkylcarboxylates; and
**■** polymeric surfactants - including those based upon homopolymers, block copolymers, and graft copolymers.

Suitable groups of anionic surfactants include alkyl ether phosphates, alkyl phenol ether phosphates, alkyl phenol ether sulphates, alkyl naphthalene sulphonates, condensed naphthalene sulphonates, aromatic hydrocarbon sulphonic acids, salts and blends, fatty alcohol sulphates, alkyl ether carboxylic acids and salts, alkyl ether sulphates, mono-alkyl sulphosuccinamates, di-alkyl sulphosuccinates, alkyl phosphates, alkyl benzene sulphonic acids and salts, and alpha olefin sulphonates.

Anionic surfactants, by way of example, may further include sodium dodecyl sulphate (SDS), ammonium lauryl sulphate, and other alkyl sulphate salts, sodium laureth sulphate, also known as sodium lauryl ether sulphate (SLES), and alkyl benzene sulphonate.

Suitable groups of cationic surfactants include alkyl dimethylamines and alkyl amidopropylamines, quaternary amine ethoxylates, and quaternary ammonium compounds.

Cationic surfactants, by way of example, may further include cetyl trimethylammonium bromide (CTAB - hexadecyl trimethyl ammonium bromide), and other alkyltrimethylammonium salts, cetyl pyridinium chloride, polyethoxylated tallow amine (POEA), and benzalkonium chloride,

Suitable groups of non-ionic surfactants include alkyl polysaccharides, alkylamine ethoxylates, amine oxides, block copolymers, castor oil ethoxylates, ceto-oleyl alcohol ethoxylates, ceto-stearyl alcohol ethoxylates, decyl alcohol ethoxylates, dinonyl phenol ethoxylates, dodecyl phenol ethoxylates, end-capped ethoxylates, ethoxylated alkanolamides, ethylene glycol esters, fatty acid alkanolamides, fatty alcohol alkoxylates, lauryl alcohol ethoxylates, mono-branched alcohol ethoxylates, nonyl phenol ethoxylates, octyl phenol ethoxylates, random copolymer alkoxylates, sorbitan ester ethoxylates, stearic acid ethoxylates, synthetic alcohol ethoxylates, tall oil fatty acid ethoxylates, and tallow amine ethoxylates.

Non-ionic surfactants, by way of example, may further include alkyl poly(ethylene oxide), alkyl polyglucosides, including octyl glucoside and decyl maltoside, fatty alcohols including cetyl alcohol and oleyl alcohol, cocamide MEA, cocamide DEA, and cocamide TEA.

Suitable groups of zwitterionic surfactants include alkyl ampho (di) acetates, amido betaines, and alkyl betaines.

Zwitterionic surfactants, by way of example, may further include dodeycl betaines, dodecyl dimethylamine oxide, cocamidopropyl betaines, and coco ampho glycinate.

Suitable groups of polymeric surfactants include modified polysaccharides, polycarboxylates, polyoxamers, and those derived by ethylene oxide condensation with the reaction product of propylene oxide and ethylene diamine.

Polymeric surfactants, by way of example, may further include polyethylene glycol/poly-12-hydroxy stearic acid copolymers, and methoxy ethylene glycol methacrylate.

Surfactants are available commercially from, for example, Huntsman Performance Products. Additionally, suitable products comprising surfactants may be used, such as domestic washing up liquid sold under the trade name Fairy.

The surfactants may be used alone or in any suitable combination.

The at least one surfactant may be present in the range 30 wt.% to 0.001 wt.% of the control formulation. More preferably, the surfactant is present in the range 20 wt.% to 0.01 wt.%. Most preferably, the surfactant is present in the range 10 wt.% to 0.1 wt.%.

The at least one gum may be any suitable gum or gelling agent. It will be understood that this would include any suitable synthetic or natural gum, or gelling agent which capable of acting as a thickening agent.

Suitable gums may include, by way of example, those based upon arabic gums, guar gums, xantham gums, pectin gums, or locust bean gums (carob gum/carubin).

The gum may be used alone or in any suitable combination.

The at least one gum may be present in the range 10 wt.% to 0.1 wt.% of the control formulation. Preferably, the gum is present in the range 8 wt.% to 0.1 wt.%.

The control formulation may comprise at least one fatty acid. The fatty acid may be any suitable fatty acid.

Suitable fatty acids include those described as saturated fatty acids and also those described as unsaturated fatty acids.

Saturated fatty acids do not contain any double bonds or other functional groups along the chain. The term "saturated" refers to hydrogen, in that all carbons (apart from the carboxylic acid [-COOH] group) contain as many hydrogens as possible.

Specific examples of saturated fatty acids include butyric acid, lauric (dodecanoic acid), myristic (tetradecanoic acid), palmitic (hexadecanoic acid), stearic (octadecanoic acid), arachidic (eicosanoic acid), castor oil, glyceryl trioleate, and andcinoleic acid.

Unsaturated fatty acids are of similar form to saturated fatty acids, except that one or more alkenyl functional groups exist along the chain, with each alkene substituting a singly-bonded " -CH₂-CH₂-" part of the chain with a doubly-bonded "-CH=CH-" portion.

Suitable groups of unsaturated fatty acids, by way of example, include those described as omega-3 fatty acids, omega-6 fatty acids, and omega-9 fatty acids.

Specific examples of unsaturated fatty acids include palmitoleic acid, alpha-linolenic acid, docosahexaenoic acid, eicosapentaenoic acid, linoleic acid, arachidonic acid, oleic acid, and erucic acid.

The fatty acids may be used alone or in any suitable combination.

The at least one fatty acid may be present in the range 25 wt.% to 0.1 wt.% of the control formulation. More preferably, the fatty acid is present in the range 20 wt.% to 0.1 wt%. Most preferably, the fatty acid is present in the range 8 wt.% to 0.1 wt.%.

Without wishing to be unduly bound by theory, it has been found that the benefits of using fatty acids may provide for protection of the active agent in the control formulation from degradation, for example by naturally present ultraviolet light and other weathering. The protection of the active agent from degradation allows for an increased lifespan of the control formulation both in storage ptior to application, and once applied to a desired site.

The control formulation may also comprise water.

The water, if present, may be present in the range 90 wt.% to 40 wt.% of the control formulation. More preferably, the water is present in the range 80 wt.% to 50 wt.%.

The control formulation may comprise at least one antifreeze compound. It will be understood that this would include any suitable compound which lowers the freezing point of water.

Suitable antifreeze compounds include those based upon alcohols and glycols.

Antifreeze compounds which may be used, by way of example, include methanol, ethylene glycol, propylene glycol, and dipropylene glycol.

The antifreeze, if present, may be preset in the range 50 wt.% to 10 wt.% of the control formulation.

The control formulation may also include additional ingredients, such as modifying agents. The modifying agents, for example, may be selected from pigments/dyes, UV absorbers, UV stabilisers, anti-fungal compounds, anti-bacterial compounds, disinfectants, inorganic carriers, organic carriers, antioxidants, perfumes, and viscosity modifiers.

The control formulation may comprise at least one fragrance. Any suitable fragrance may be used in the control formulation.

The components of the invention are mixed in the desired proportions to provide a control formulation which has odour control properties. Additionally, the control formulation can provide bird repellent and bird control properties.

Thus, according to a second aspect of the present invention there is provided a method of making a control formulation which comprises mixing;
at least one active agent;
at least one solvent;
at least one surfactant; and
at least one gum,
wherein the active agent is an anthranilic acid derivative, acetophenone derivative, veratroyl derivative, cinnamic acid derivative, or alkyl phenyl acetate derivative, and the formulation is adapted for odour control and bird control.

One particularly preferred combination of the components of the present invention is listed below.
methyl anthranilate, orange terpene oil, pine oil, xantham gum, water, surfactant (from domestic washing up liquid), arabic gum, guar gum, pectin, locust bean gum, and saturated and unsaturated fatty acids.

The control formulation may be applied in a liquid, aerosol, or dry form to the desired site or field. Liquid and aerosol application are preferred.

Application may be by any suitable method, such as liquid pumps (manual and power operated), liquid atomizers (hydraulic energy, gaseous energy, and centrifugal energy), and dry application.

It is envisaged that the improved odour control and bird control formulation of the present invention will find application in control of odours and birds on waste sites, and in particular landfill waste sites.

Thus, according to a third aspect of the invention there is provided the use of a control formulation in accordance with the first aspect for the reduction of odours at a desired area.

Thus, according to a fourth aspect of the invention there is provided the use of a control formulation in accordance with the first aspect for the reduction and control of birds at a desired area

Suitable desires areas include, by way of example, landfill waste sites, or crop fields.

Without wishing to be unduly bound by theory, it has been found that the benefits of the invention may be conferred due to the combination of the active agent with the surfactant, gum, and solvent such that the control formulation is effective as both a bird repellent and odour controller. The control formulation also allows for easy application to the desired site with both increased effectiveness and reduced side effects on humans and plant life in the area when compared to prior formulations.

All of the features described herein may be combined with any of the above aspects, in any combination.

In order that the present invention may be more readily understood, reference will now be made, by way of example, to the following description.

A specific example of a control formulation is listed in table 1.

**Table 1**

| **Component** | **Percentage (%)** | **Amount in 250m³ Solution (m³)** |
|---|---|---|
| Methyl anthranilate | 16.2 | 40.5 |
| Water | 70 | 175 |
| Orange terpene | 11.5 | 28.75 |
| Xanthan Gum | 0.4 | 1 |
| Guar gum | 0.2 | 0.5 |
| Surfactant (in the form of Fairy washing up liquid) | 0.4 | 1 |
| Vegetable oil | 1.2 | 3 |
| Fatty acid | 0.1 | 0.25 |

It is envisaged that the gums are added to the vegetable oil in a first step to form an emulsion. The resulting emulsion can then be added to the other components of the control formulation.

The control formulation can then be used in a spray delivery apparatus, such as a manual or power operated liquid pump. The formulation would then be sprayed over the area intended to be treated, such as a landfill waste site. The spray could be reapplied as desired to ensure that the benefit of odour control and bird control/bird repellent is maintained.

It is envisaged that a 250m³ formulation as detailed in Table 1 would be of typical volume to treat a suitable landfill waste site.

It is observed that when the control formulation is applied to a landfill waste site in the form of an aerosol spray the total number of birds attracted to, and landing on, the site was reduced. The application of the control formulation to the landfill site also significantly reduces the odours which emanate from the site. The control formulation on application to a landfill site therefore exhibited effects on reducing both bird numbers and reducing odours.

It was also observed that the control formulation had little if any significant effect on surrounding plant life.

The formulation detailed in Table 1 is used in a water diluted form. However, the control formulation may also be formulated as either concentrate or ready-to-use solution which is not toxic to humans, birds and other wildlife, or plants.

The control formulation is able to be used and applied to areas other than landfill waste sites, such as crop fields or other land where there is a desire to reduce both odours and to control or reduce the presence of birds.

It is to be understood that the invention is not to be limited to the details of the above embodiments, which are described by way of example only. Many variations are possible.

## Claims

1. A control formulation, wherein the formulation comprises;
at least one active agent;
at least one solvent;
at least one surfactant; and
at least one gum,
wherein the active agent is an anthranilic acid derivative, acetophenone derivative, veratroyl derivative, cinnamic acid derivative, or alkyl phenyl acetate derivative, and the formulation is for odour control or bird control.

2. A control formulation according to claim 1, wherein the at least one active agent is selected from methyl anthranilate, methyl phenyl acetate, ethyl phenyl acetate, ortho-amino acetophenone, 2-amino-4,5-dimethyl acetophenone, veratroyl amine, dimethyl anthranilate, cinnamic aldehyde, cinnamamide, cinnamic acid, and combinations thereof

3. A control formulation according to either claim 1 or claim 2, wherein the at least one active agent is methyl anthranilate.

4. A control formulation according to any preceeding claim, wherein the at least one active agent is present in the range 30 wt.% to 2 wt.% of the control formulation.

5. A control formulation according to any preceeding claim, wherein the at least one solvent comprises at least one oil component selected from terpenes or naturally occurring extracted oils.

6. A control formulation according to claim 5, wherein the at least one oil component is selected from hemiterpenes, monoterpenes, sesquiterpenes, diterpenes, sesterterpenes, triterpenes, tetraterpenes, polyterpenes, tree extracted oils, vegetable oils, or any combination thereof.

7. A control formulation according to any preceeding claim, wherein the at least one solvent is present in the range 40 wt.% to 2 wt.% of the control formulation

8. A control formulation according to any preceeding claim, wherein the at least one surfactant is selected from anionic, cationic, non-ionic surfactants, zwitterionic (amphoteric) surfactants, and polymeric surfactants.

9. A control formulation according to any preceeding claim, wherein the at least one surfactant is present in the range 30 wt.% to 0.001 wt.% of the control formulation.

10. A control formulation according to any preceeding claim, wherein the at least one gum is selected from arabic gums, guar gums, xantham gums, pectin gums, or locust bean gums (carob gum/carubin).

11. A control formulation according to any preceeding claim, wherein the at least one gum is present in the range 10 wt.% to 0.1 wt.% of the control formulation.

12. A control formulation according to any preceeding claim, wherein the control formulation comprises at least one fatty acid.

13. A control formulation according to claim 12, wherein the fatty acid is selected from butyric acid, lauric (dodecanoic acid), myristic (tetradecanoic acid), palmitic (hexadecanoic acid), stearic (octadecanoic acid), arachidic (eicosanoic acid), castor oil, glyceryl trioleate, and andcinoleic acid.

14. A control formulation according to either claim 12 or claim 13, wherein the at least one fatty acid is present in the range 25 wt.% to 0.1 wt.% of the control formulation.

15. A control formulation according to any preceeding claim, wherein the control formulation comprises at least one antifreeze compound.

16. A control formulation according to any preceeding claim, wherein the control formulation comprises additional ingredients selected from pigments/dyes, UV absorbers, UV stabilisers, anti-fungal compounds, anti-bacterial compounds, disinfectants, inorganic carriers, organic carriers, antioxidants, perfumes, and viscosity modifiers.

17. A control formulation according to any preceeding claim, wherein the control formulation comprises at least one fragrance.

18. A method of making a control formulation which comprises mixing;
at least one active agent;
at least one solvent;
at least one surfactant; and
at least one gum,
wherein the active agent is an anthranilic acid derivative, acetophenone derivative, veratroyl derivative, cinnamic acid derivative, or alkyl phenyl acetate derivative, and the formulation is adapted for odour control and bird control.

19. Use of a control formulation in accordance with claim 1 for the reduction of odours.

20. Use of a control formulation in accordance with claim 1 for the deterring birds.
